(19) Europäisches Patentamt
European Patent Office
Office européen des brevets

(11) Publication number: **0 031 686**
**B1**

(12) **EUROPEAN PATENT SPECIFICATION**

(45) Date of publication of patent specification: **29.10.86**

(21) Application number: **80304610.1**

(22) Date of filing: **19.12.80**

(51) Int. Cl.$^4$: **C 07 D 317/30,**
C 07 D 319/06,
C 07 C 103/38, A 01 N 43/08,
A 01 N 43/16, A 01 N 37/18

# (54) Antidotal compounds, their preparation and their use with herbicides.

(30) Priority: **26.12.79 US 106433**
**26.12.79 US 106434**

(43) Date of publication of application:
**08.07.81 Bulletin 81/27**

(45) Publication of the grant of the patent:
**29.10.86 Bulletin 86/44**

(84) Designated Contracting States:
**AT BE CH DE FR GB IT LI NL**

(56) References cited:
**US-A- 113 464**
**US-A-3 859 308**
**US-A-3 894 046**
**US-A-3 894 048**
**US-A-3 900 497**
**US-A-3 948 950**
**US-A-4 116 670**
**US-A-4 137 070**
**US-A-4 155 745**

**The file contains technical information submitted after the application was filed and not included in this specification**

(73) Proprietor: **PPG INDUSTRIES, INC.**
**One PPG Place**
**Pittsburgh Pennsylvania 15272 (US)**

(72) Inventor: **Rinehart, Jay Kent**
**634 Ecton Road**
**Akron, OH 44303 (US)**

(74) Representative: **Shipton, Gordon Owen et al**
**W.P. THOMPSON & CO. Coopers Building**
**Church Street**
**Liverpool L1 3AB (GB)**

(56) References cited:
**J. Agric. Food Chem., 23, 4(1975), pp. 821-822**
**Agric and Food Chemistry, 5, 1(1957), pp. 530-532**

**Chemistry and Action of Herbicide Antidotes, Academic Press (1978), p. 35-61**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to N-(2,2-dialkoxyethyl)-N-(prop-2-en-1-yl)-2,2-dichloroacetamides and to N-(optionally substituted 1,3-dioxolan- 2-ylmethyl)-N-(prop-2-en-1-yl)-2,2-dichloroacetamides, their use as antidotes, herbicidal compositions containing these compounds, and methods of preparation of these compounds.

US—A—4,113,464 (Stach et al) discloses compounds of the formula:

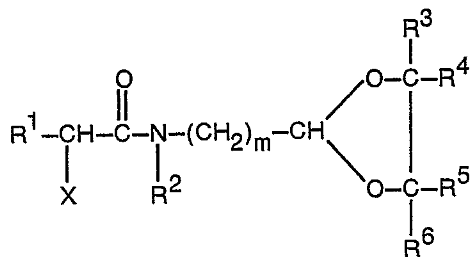

wherein $R^1$ is selected from the group consisting of hydrogen and alkyl; X is halogen; $R^2$ is selected from the group consisting of alkyl, alkenyl and alkynyl; m is the integer 1 or 2; and $R^3$, $R^4$, $R^5$ and $^6$ are each selected from the group consisting of hydrogen and alkyl. It also discloses herbicidal compositions utilizing these compounds. However, these monochloro compounds are not described to be antidotally effective.

US—A—4,116,670 discloses compounds having the formula

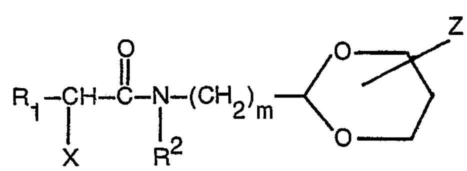

wherein $R^1$ is selected from the group consisting of hydrogen and alkyl; X is halogen; $R^2$ is selected from the group consisting of alkyl, alkenyl and alkynyl; n is the integer 1 or 2; Z is alkyl; and m is an integer from 0 to 4 which are useful as herbicides.

US—A—4,155,745 discloses compounds having the following structural formula

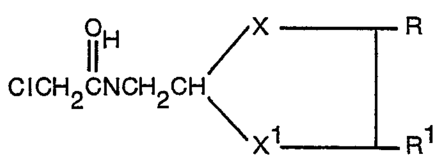

wherein R and $R^1$ independently are:

(a) alkyl having 1 to 4 cabon atoms,
(b) haloalkyl having 1 to 4 carbon atoms,
(c) phenyl or
(d) hydrogen X is oxygen or sulfur, and $X^1$ is oxygen or sulfur which are useful as herbicides.

US—A—4,137,070 discloses a herbicidal composition comprising an active thiolcarbamate herbicide compound and an antidotally effective amount of an antidote compound having the formula

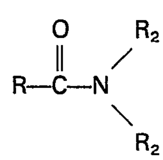

wherein R is haloalkyl containing from 1 to about 6 carbon atoms, inclusive; and $R^1$ and $R^2$ are alkenyl containing from 2 to about 12 carbon atoms, inclusive; said compound being antidotally active with said thiolcarbamate herbicide compound and wherein said compound is present in an amount varying between about 0.0001 to 30 parts by weight for each part by weight of the active herbicidal compound. A

dichloroacetamide specifically mentioned is N,N-diallyl dichloroacetamide but this differs from the cyclic diacetal compounds of the present invention by the absence of a dioxolan nucleus. A monochloroacetamide specifically mentioned is N-(2,2-dimethoxyethyl) chloroacetamide but this differs from the non-cyclic diacetal compounds of the present invention in having only one chlorine atom in the molecule and by the absence of the prop-2-en-1-yl radical.

GB—A—1,396,941 and GB—A—1,396,942 (divided out of GB—A—1,397,941) are each based on the United States Patent Application which matured into US—A—4,137,070. The remarks made above in relation to the US—A—4,137,070 are relevent to GB—A—1,396,941 and GB—A—1,396,942.

Hamm and Speziale [Journal of Agricultural and Food Chemistry, *5*, 1, (1957) Pages 30—32] states that monochloroacetamides have greater herbicidal activity than dichloroacetamides but does not state that the monochloro compounds would therefore exhibit antidotal activity.

The paper by Pallos et al in the Journal of Agricultural and Food Chemistry [*23*, 4, (1975) pages 821—822] forms part of "Chemistry and Action of Herbicidide Antidotes" [Academic Press (1978)] which is a compilation of papers on this subject. Pallos et al state that dichloroacetamides have greater antidotal activity than monochloroacetamides but Table 9 in an article by Stephenson and Chang on page 51 of the same compilation clearly discloses that N,N-diallylmonochloroacetamide and N,N-diallyldichloroacetamide show an identical extent of corn injury.

In one embodiment, this invention relates to N-(2,2-dialkoxy-ethyl)-N-prop-2-en-1-yl-2,2-dichloroacetamides represented by the formula II

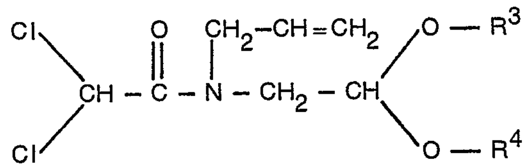

II

wherein:

$R^3$ and $R^4$ are alkyl of up to 4 carbon atoms.

In a second embodiment, this invention relates to N-(optionally substituted 1,3-dioxolan -2-ylmethyl)-N-(prop-2-en-1-yl)-2,2-dichloroacetamides represented by the Formula (I)

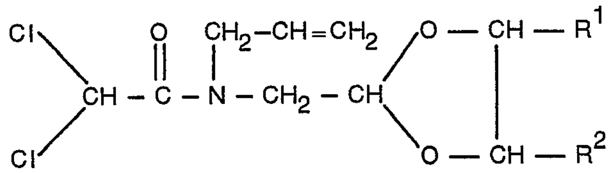

I

wherein

$R^1$ and $R^2$ are independently hydrogen or methyl.

The compounds of this invention, as represented by Formulae (I) and (II), have been found eminently well-suited for protecting growing crops, for example, corn, from the phytotoxic effects of active herbicides, particularly S-alkyl thiocarbamate herbicides. Compounds of this invention that have been found to exhibit especially efficacious long-term, antidotal effects i.e., lasting up to three weeks or more after application, are N-(2,2-dimethoxyethyl)-N-(prop-2-en-1-yl)-2,2-dichloroacetamide, i.e., a compound of Formula (II) wherein $R^3$ and $R^4$ are methyl; and N-(1,3-dixolan-2-ylmethyl)-N-(prop-2-en-1-yl)-2,2-dichloroacetamide, i.e., a compound of Formula (I) wherein $R^1$ and $R^2$ are hydrogen.

The compounds represented by Formula (II) may be prepared by reacting an appropriately substituted N-(2,2-dialkoxyethyl)amine of the formula (IV)

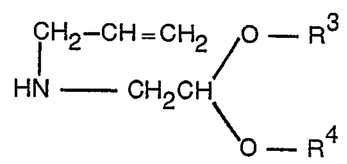

IV

wherein $R^3$ and $R^4$ are as defined hereinabove with an at least equimolar amount of dichloroacetyl chloride in the presence of an acid acceptor such as tertiary amine, for example, triethylamine. Preferably the reaction is conducted in the presence of an inert organic solvent, for example, methylene chloride or

benzene. The reaction is typically conducted at a temperature ranging from 0°C to ambient, i.e., 20°C to 23°C, for a time sufficient to obtain the desired extent of conversion which may range from a few minutes to a few hours. At the completion of the reaction, the reaction mixture is washed with dilute acid, dried and stripped of solvent. The product is typically obtained in liquid, substantially pure, form and generally does not require further purificiation.

The cyclic diacetal compounds represented by Formula (I) may conveniently be prepared by reacting a diacetal compound of Formula (II) with an at least equimolar amount of a diol represented by the Formula (III)

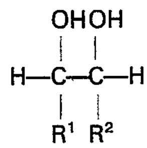

III

wherein $R^1$ and $R^2$ are as defined hereinabove, under anhydrous conditions and in the presence of an inorganic or organic acid catalyst, such as, for example, sulfuric acid or p-toluene sulfonic acid. The reaction is typically conducted at elevated temperature, e.g., 90°C to 120°C, for a time sufficient to obtain the desired extent of conversion, after which the reaction product is washed, dried, and worked-up in known fashion.

The following Examples 1 and 2 are illustrative of the preparation of certain specific antidotal compounds of this invention.

### Example 1

Preparation of N-(2,2-dimethoxyethyl))-N-(prop-2-en-yl)-2,2-dichloroacetamide

A three-neck flask was placed in an ice bath and charged with 2.9 grams (0.02 mole of N-(prop-2-en-1-yl)-N-(2,2-dimethoxyethyl)amine, and 2.02 grams (0.02 mole) of triethylamine in 100 milliliters of methylene chloride. To this stirred mixture, a solution of 2.95 grams (0.02 mole) of dichloroacetyl chloride in 5 milliliters of methylene chloride was added dropwise over a period of 15 minutes. The reaction mixture was stirred for one-half hour, removed from the ice bath, and stirred for an additional three hours, by which time the reaction mixture had reached ambient temperature.

The reaction mixture was then washed with a 50 milliliter portion of 10 percent aqueous hydrochloric acid solution, dried over magnesium sulfate, and concentrated on a rotary evaporator at a temperature not in excess of 50°C., yielding 4.96 grams of a pale pink liquid identified by NMR spectroscopy as N-(2,2-dimethoxyethyl)-N-prop-2-en-1-yl)-2,2-dichloroacetamide.

### Example 2

Preparation of N-(1,3-dioxolan-2-ylmethyl)-N-(2-propenyl)-2,2-dichloroacetamide

To a round-bottom flask was charged 15.0 grams (0.059 mole) of N-(2,2-dimethoxyethyl)-N-(2-propenyl)-2,2-dichloroacetamide, prepared as described in Example 1, 6.2 grams (0.1 mole) of ethylene glycol and two crystals of p-toluene sulfonic acid. The reaction mixture was heated for 4 hours at about 110°C., the methanol generated being distilled off. The reaction mixture was then cooled, poured into 100 milliliters of methylene chloride, and washed once with a 50-milliliter portion of 10 percent aqueous sodium hydroxide solution and washed twice with two 100-milliliter portions of water. The washed reaction mixture was dried over sodium sulfate and concentrated on a rotary evaporator yielding 13.18 grams of a dark liquid identified by NMR spectroscopy as N-(1,3-dioxolan-2-ylmethyl)-N-(prop-2-en-1-yl)2,2-dichloroacetamide.

The mode of synthesis of specific compounds of this invention has been illustrated in some detail by the foregoing Examples; but it is to be understood that any compound contemplated to be within the scope of this invention may be prepared by those skilled in the art simply by varying the choice of starting materials and use of the illustrated techniques or other suitable techniques.

The compounds prepared as described in Example 1 and 2 as well as other compounds within the scope of this invention are useful in reducing phytotoxic damage caused by active herbicides to growing crops, especially corn. The active herbicides which tend to phytotoxically damage corn, when used in herbicidally effective amount against weeds growing among the corn crop, are S-alkyl thiocarbamate-type herbicides. Exemplary of some S-alkyl thiocarbamate-type herbicides are S-ethyl diethylthiocarbamate, S-ethyl diisobutylthiocarbamate, S-ethyl dipropylthiocarbamate, and S-propyl dipropylthiocarbamate; chloroacetanilide-type herbicides.

The S-alkyl thiocarbamate herbicides are typically incorporated into the soil or growth medium prior to crop seeding, which technique is commonly referred to as "pre-plant incorporation." The antidotal compounds of this invention may be separately incorporated into the soil but are preferably formulated or composited with the S-alkyl thiocarbamate herbicide. Sufficient of the antidotal compounds of this invention are present such that the weight ratio of herbicide to compound of this invention is in the range of 18:1 to 6:1, the herbicide, of course, being present in herbicidal amount which, depending on the weed species, may vary over a wide range. Typically the herbicide is used in amounts ranging from 0.56 or less kilogram per hectare (0.5 pound per acre) to 11.2 or more kilograms per hectare (10 pounds per acre).

It is to be further understood that mixtures of S-alkyl thiocarbamate herbicides as well as mixtures of compounds of this invention may be used. In additiion, the herbicide-antidotal compound formulations of this invention may contain other agronomically acceptable adjuvants such as inert carriers, herbicides other than S-alkyl thiocarbamates, or other commonly used agricultural compounds, for example, pesticides, stabilizers, fertilizers, soil life extending agents, and the like.

The compounds of this invention, whether used as such or in formulation with other materials, may be applied to the soil in the form of dusts, granules, wettable powders, solutions suspensions aerosols, emulsions, dispersions, or the like in a manner well-known to the art.

The following Example 3 is illustrative of the antidotal effect exhibited by the compounds of this invention in protecting Funk's G-4288 hybrid field corn from the phytotoxic damage caused by a commercial herbicide, namely, S-ethyl dipropylthiocarbamate (commonly referred to as EPTC).

Example 3

(a) Pulverized, sandy loam topsoil and coarse, washed cement sand screened to pass a 0.475 centimeter mesh screen were mixed in a weight ratio of three parts of soil to one part of sand, and the mix was pasteurized with live steam to kill any plants, pathogenic organisms, and natural weed seed populations.

Shallow containers, commonly referred to as "flats", were filled within 0.95 centimeter of the top with the pasteurized soil mixture, levelled but not firmed, and were passed under a sprayer equipped with a fixed fan nozzle suspended over a movable belt with speed adjustment. The fan nozzle operated at about 2800 grams per square centimeter air pressure, delivering 25 milliliters of distilled water in 4.6 seconds. At 25.4 centimeters above the soil surface, a 45.7 centimeter wide spray pattern was applied. The belt speed was adjusted so as to travel 76.2 centimeters per revolution in 8.3 seconds. The soil test containers passed under the nozzle within 4.6 seconds. The area covered in this time was 0.63 square meters. The volume of liquid delivered was equivalent to 400 liters per hectare and 0.7029 gram of a 100 percent active test compound per 0.63 square meter was equivalent to an application rate of 11.2 kilograms of active test compound per hectare.

The carrier solvent used for the S-alkyl thiocarbamate, i.e., EPTC, and the particular antidotal compound was a 90:8:2 volume/volume mixture of acetone:methanol:dimethylformamide. After the test container was sprayed with the test compound or mixture of compounds, it was immediately emptied into a clean plastic bag, the top secured, and the contents thoroughly mixed by hand agitation of the plastic bag, and the contents were then emptied back into the test container. The soil was then leveled, firmed, and seeded with Funk's G-4288 Hybrid Field Corn, and the soil mixture covered with a 2.54 centimeter layer of pasteurized screened sand. The treated flats were then transferred to the plant growth room, where they were lightly watered overhead as required to ensure growth. The plant growth room was illuminated with a light intensity that averaged 27,000 lumens per square meter at the growth level. The growth room was maintained at 29°C. to 30°C. during the 16-hour photo period, and 20°C to 23°C. at night. The relative humidity of the growth room when less than one-third full averaged from 50 to 55 percent.

The treated flats were allowed to grow and injury, if any, to the corn was determined by visual inspection periodically thereafter. Injury was noted as reduction in growth, and/or as hormonal distortion as compared with an untreated control.

This hormonal injury is manifested as a distortion of the growing apex of the stem due to the failure of the developing leaves to unfurl from the growing shoot. Continued apical growth results in a compaction and distortion of the subapical tissues until a break is caused in the surrounding leaf tissue. Hormonal injury may occur in the traditional seedling· stages of growth when high concentrations of S-alkyl thiocarbamate has been applied or at later growth stages with lower concentrations of the herbicide. The distortion may not appear until the growing apex of the stem is above the soil line or may not appear until the apex divides and begins formation of the pistillate inflorescens. Hormal injury may also weaken the prop foot system so as to cause the whole plant to collapse of its own weight.

(b) When EPTC (S-ethyl dipropylthiocarbamate) was applied by pre-plant incorporation as described in paragraph (a) at a rate of 6.72 kilograms per hectare with no antidotal compound added, as described, the corn showed a 90 percent injury as indicated by a reduction in growth and hormonal distortion after 14 days, 25 days, 28 days, and 42 days. In addition, after 42 days, necrosis also developed to contribute to the injury of the corn crop.

(c) When EPTC at a rate of 6.72 kilograms per hectare and N-(2,2-dimethoxyethyl)-N-(2-propenyl)-2,2-dichloroacetamide (prepared as described in Example 1) at a rate of 0.56 kilograms per hectare, that is, a ratio of 12 parts by weight of EPTC to one part by weight of antidotal compound, were applied by pre-plant incorporation as described in paragragh (a), the corn crop, after 42 days, was completely healthy and normal and showed no signs of growth reduction or hormonal distortion.

(d) When EPTC at a rate of 7.72 kilograms per hectare and N-(1,3-dioxolan-2-ylmethyl)-N-(2-propenyl)-2,2-dichloroacetamide (prepared as described in Example 2) at 1.12 kilograms per hectare, that is, a rate of 6 parts by weight of EPTC to one part by weight of antidotal compound, were applied by pre-plant incorporation as described in paragraph (a), the corn crop, after 42 days, was completely healthy and normal and showed no signs of growth reduction or hormonal distortion.

**Claims**

1. A compound characterised by the formula (I)

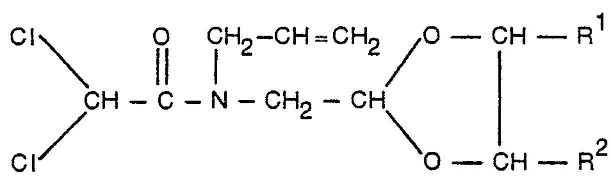

I

wherein:

$R^1$ and $R^2$ are independently hydrogen or methyl.

2. A compound as claimed in claim 1 characterised in that $R^1$ and $R^2$ are hydrogen.

3. A composition containing a herbicidally effective amount of S-alkyl thiocarbamate herbicide and characterised by containing an antidotally effective amount of a compound defined in claim 1 or 2, wherein the weight ratio of herbicide to antidotal compound ranges from 18:1 to 6:1.

4. A method of controlling weed growth among crops characterised by controlling said weeds with a composition as claimed in claim 3.

5. A process for preparing a compound of claim 1, characterised by reacting a diacetal of the formula (II)

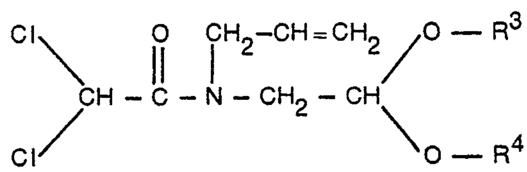

II

under anhydrous conditions and in the presence of an acid catalyst, with at least an equimolar amount of a diol of the formula (III)

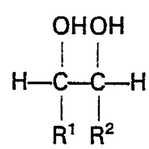

III

wherein:

$R^1$ and $R^2$ are as defined in claim 1 and $R^3$ and $R^4$ are each alkyl of up to 4 carbon atoms.

6. A compound characterised by the formula (II):

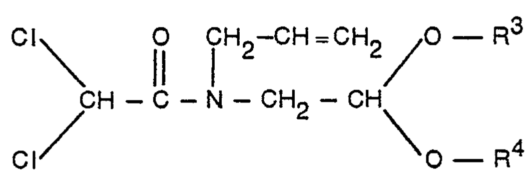

II

wherein:

$R^3$ and $R^4$ are each alkyl of up to 4 carbon atoms.

7. A compound as claimed in claim 6, characterised in that $R^3$ and $R^4$ are each methyl.

8. A composition characterised by containing a herbicidally effective amount of S-alkyl thiocarbamate herbicide and an antidotally effective amount of a compound defined in claim 6 or 7, wherein the weight ratio of herbicide to antidotal compound ranges from 18:1 to 6:1.

9. A method of controlling weed growth among crops, characterised by controlling said weeds with a composition as claimed in claim 8.

10. A process for preparing a compound of claim 6, characterised by reacting a substituted N-(2,2-dialkoxyethyl)amine of the formula (IV)

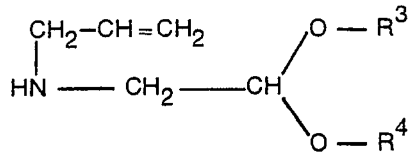

IV

wherein $R^3$ and $R^4$ are as defined in claim 6, with an at least equimolar amount of dichloroacetyl chloride in the presence of an acid acceptor.

**Patentansprüche**

1. Eine Verbindung, gekennzeichnet durch die Formuel (I)

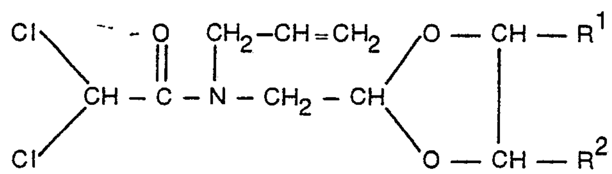

I

worin

$R^1$ and $R^2$, unabhängig voneinander, Wasserstoff oder Methyl bedeuten.

2. Eine Verbindung wie in Anspruch 1 beansprucht, dadurch gekennszeichnet, daß $R^1$ und $R^2$ Wasserstoff sind.

3. Eine Zusammensetzung, enthaltend eine herbizid wirksame Menge an S-Alkylthiocarbamat-Herbizid und gekennzeichnet durch einen Gehalt an einer antidotisch wirksamen Menge einer Verbindung, wie sie in Anspruch 1 oder 2 definiert ist, wobei das Gewichtsverhältnis von Herbizid zu antidotischer Verbindung im Bereich von 18:1 bis 6:1 liegt.

4. Verfahren zur Bekämpfung des Unkraut-Wachstums in Feldkulturen, gekennzeichnet durch die Bekämpfung der genannten Unkräuter mittels einer Zusammensetzung, wie sie in Anspruch 3 beansprucht ist.

5. Verfahren zur Herstellung einer Verbindung gemaß Anspruch 1, gekennzeichnet durch die Reaktion eines Diazetals der Formel (II)

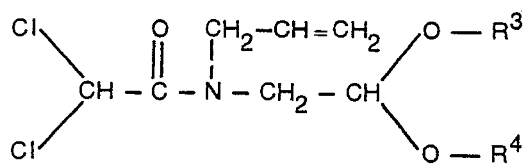

II

unter wasserfreien Bedingungen und in Gegenwart eines Säurekatalysators mit mindestens einer äquimolaren Menge eines Diols der Formel (III)

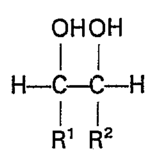

III

worin

$R^1$ und $R^2$ die in Anspruch 1 angegebene Bedeutung haben, und $R^3$ und $R^4$ jeweils eine Alkylgruppe bis zu 4 C-Atomen darstellen.

6. Eine Verbindung, gekennzeichnet durch die Formel (II)

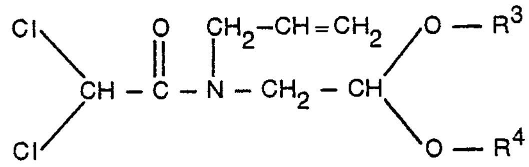

II

worin
$R^3$ and $SR^4$ jeweils eine Alkylgruppe bis zu 4 C-Atomen darstellen.

7. Eine Verbindung wie in Anspruch 6 beansprucht, dadurch gekennzeichnet, daß sowohl $R^3$ als auch $R^4$ Methyl ist.

8. Eine Zusammensetzung, gekennzeichnet durch einen Gehalt an einer herbizid wirksamen Menge an S-Alkylthiocarbamat-Herbizid und einer antidotisch wirksamen Menge einer Verbindung wie sie in Anspruch oder 7 definiert ist, wobei das Gewichtsverhältnis von Herbizid zu antidotischer Verbindung im Bereich von 18:1 bis 6:1 liegt.

9. Verfahren zur Bekämpfung des Unkrautwachstums in Feldkulturen, gekennzeichnet durch Bekämpfung der genannten Unkräuter mittels einer Zusammensetzung, wie sie im Anspruch 8 beansprucht ist.

10. Ein Verfahren zur Herstellung einer Verbindung gemäß Anspruch 6, gekennzeichnet durch die Reaktion eines substituierten N-(2,2-Dialkoxyethyl)amins der Formel (IV)

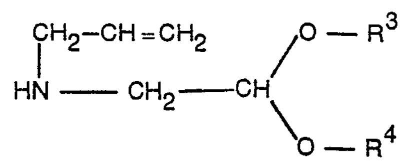

IV

worin $R^3$ and $R^4$ die in Anspruch 6 angegebene Bedeutung haben, mit zumindest einer äquimolaren Menge an Dichloracetylchlorid in Gegenwart eines Säureakzeptors.

**Revendications**

1. Composé caractérisé par la formule (I)

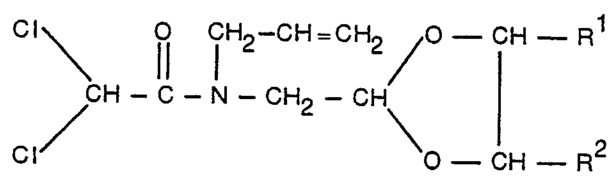

I

dans laquelle:
$R^1$ et $R^2$ représentent, indépendamment, un atome d'hydrogène ou un groupe méthyle.

2. Composé selon la revendication 1, caractérisé en ce que $R^1$ et $R^2$ représentent chacun un atome d'hydrogène.

3. Composition contenant une quantité, efficace comme herbicide, d'un herbicide de type thiocarbamate de S-alkyle et caractérisée en ce qu'elle contient une quantité, capable de jouer le rôle d'antidote, d'un composé défini à la revendication 1 ou 2, le rapport pondéral de l'herbicide à l'antidote allant de 18:1 à 6:1.

4. Procédé pour maîtriser la croissance des herbes parmi des plantes cultivées, caractérisé en ce qu'on maîtrise lesdites herbes à l'aide d'une composition telle que revendiquée à la revendication 3.

5. Procédé pour préparer un composé selon la revendication 1, caractérisé en ce qu'on fait réagir un diacétal de formule (II)

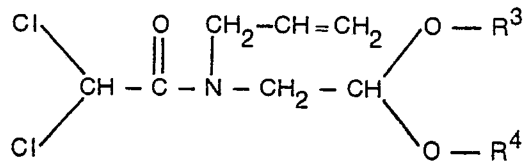

II

dans des conditions anhydres et en présence d'un catalyseur acide, avec au moins une quantité équimolaire d'un diol de formule III

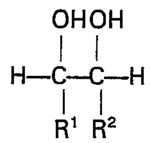

III

dans lesqwuelles:

$R^1$ et $R^1$ sont tels que définis à la revendication 1 et $R^3$ et $R^4$ représentent chacun un groupe alkyle ayant jusqu'à un maximum de 4 atomes de carbone.

6. Composé caractérisé par le formule (II)

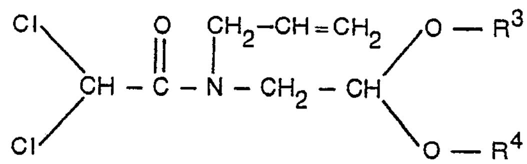

II

dans laquelle:

$R^3$ et $R^4$ représentent chacun un groupe alkyle ayant jusqu'à un maximum de e atomes de carbone.

7. Composé selon la revendication 6, caractérisé en ce que $R^3$ et $R^3$ représentent chacun un groupe méthyle.

8. Composition caractérisée en ce qu'elle contient une quantité, efficate comme herbicide d'un herbicide de type thiocarbamate de S-alkyle et une quantité, efficace à titre d'antidote, d'un composé défini à la revendication 6 ou 7, le rapport pondéral de l'herbicide à l'antidote allant de 18:1 à 6:1.

9. Procédé pour combattre la croissance d'herbes parmi des plantes cultivées, caractérisé en ce qu'on maîtrie de telles herbes à l'aide d'une composition telle que revendiquée à la revendication 8.

10. Procédé pour préparer un composer selon la revendication 6, caractérisé en ce qu'on fait réagir une N-(2,2-dialcoxyéthyl)-amine substituée de formule (IV)

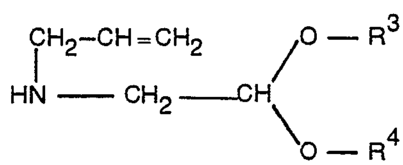

IV

dans laquelle $R^3$ et R sont tels que définis à la revendication 6, avec une quantité au moins équimolaire de chlorure de dichloroacétyle en présence d'un accepteur d'acide.